(19) Europäisches Patentamt
European Patent Office
Office européen des brevets

(11) **EP 2 562 263 A1**

(12) **EUROPEAN PATENT APPLICATION**

(43) Date of publication:
**27.02.2013 Bulletin 2013/09**

(51) Int Cl.:
**C12P 7/56** (2006.01)

(21) Application number: **12180784.6**

(22) Date of filing: **17.08.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.08.2011 JP 2011182815**

(71) Applicant: **Hitachi Plant Technologies, Ltd.**
**Tokyo**
**170-8466 (JP)**

(72) Inventors:
 • **Oka, Kenichiro**
  **Tokyo, 100-8220 (JP)**

 • **Matsuo, Toshiaki**
  **Tokyo, 100-8220 (JP)**
 • **Kamikawa, Masayuki**
  **Tokyo, 100-8220 (JP)**
 • **Okamoto, Naruyasu**
  **Tokyo, 170-8466 (JP)**
 • **Kondo, Takeyuki**
  **Tokyo, 100-8220 (JP)**

(74) Representative: **Beetz & Partner**
**Patentanwälte**
**Steinsdorfstrasse 10**
**80538 München (DE)**

(54) **Manufacturing process of purified lactic acid**

(57) An object of the present invention is to provide a manufacturing process of purified lactic acid which is a process of concentrating a lactic acid fermentation liquid at a low temperature. A manufacturing process of purified lactic acid comprising a fermentation process where a pH adjusting agent containing calcium and a microorganism are added to a sugar-containing solution to produce lactic acid in the form of calcium lactate, and a purification process where sulfuric acid is added to a solution containing calcium lactate to separate calcium ions in the form of calcium sulfate, wherein the process further comprises, prior to the step of adding sulfuric acid, a reverse osmosis membrane concentration step (140) where the solution containing calcium lactate is allowed to pass through a reverse osmosis membrane to remove water; a crystallization step (150) where the obtained concentrated liquid of calcium lactate is cooled to crystallize and remove calcium lactate; and a reverse osmosis membrane concentration step (140) where the solution from which calcium lactate has been removed is heated and allowed to pass through the reverse osmosis membrane (141a - 141c) to remove water, the crystallization step (150) and the subsequent reverse osmosis membrane concentration step (140) being repeated one or more times.

Fig. 3

**Description**

BACKGROUND OF THE INVENTION

Field of the Invention

The present invention relates to a manufacturing process of purified lactic acid.

Background Art

Lactic acid is used as the raw material for manufacturing industrial polymers, such as polylactic acid and various lactic acid products. These polymers are biodegradable and are hence very useful.

Lactic acid is produced by the fermentation of sugars. Sugars used as raw materials here include not only fermentation media containing purified glucose, etc., but often also mixed sugar systems containing galactose, fructose and various pentoses such as xylose. Mixed sugars systems are, for example, obtainable by hydrolyzing cellulose. However, these mixed sugar systems contain more impurities, such as lignin, than conventional glucose systems.

To use fermented lactic acid as a raw material for polymers, the purification step is required for impurity removal and concentration. In the step described in (WO 01/025180), a lactic acid fermentation liquid containing lactic acid in the form of calcium lactate is heated, concentrated by evaporating the water using an evaporator at a temperature of about 60°C to 150°C and adding sulfuric acid to separate calcium ion in the form of calcium sulfate, thereby obtaining purified lactic acid by solvent extraction. However, this procedure, owing to heating the fermentation liquid to a high temperature in the concentration step of lactic acid fermentation liquid, poses problems in that (1) an amount of heat is required for heating and causes high costs; (2) coloration of the solution occurs due to heat deterioration of impurities; (3) optical isomerization of lactic acid is liable to occur and the optical purity is reduced, etc. Further, the system has a drawback of lacking a reverse osmosis membrane that is compatible with the excessively high temperature to which the fermentation liquid is heated and thus failing to adopt the concentration method which uses a reverse osmosis membrane.

SUMMARY OF THE INVENTION

An object of the present invention is to provide a manufacturing process of purified lactic acid which is a process of concentrating a lactic acid fermentation liquid at a low temperature.

The present inventors conducted extensive studies to solve the above problems and found that a lactic acid purification process with reduced heat deterioration of impurities and optical isomerization of lactic acid can be achieved by, at the time of concentrating the lactic acid fermentation liquid containing lactic acid in the form of calcium lactate, first concentrating a fermentation liquid maintained at a suitable temperature using a reverse osmosis membrane, cooling the obtained concentrated liquid to crystallize calcium lactate in a solid form, heating the residual solution again and repeating the concentration process again using the reverse osmosis membrane to remove any amount of water from the fermentation liquid, and thus minimizing the heating of fermentation liquid, whereby the present invention has been accomplished.

Further, the present invention relates to a lactic acid purification process which collects calcium lactate contained in a liquid waste and prevents the lactic acid yield from being reduced by treating the liquid waste (permeate) produced from the reverse osmosis membrane concentration process with the other reverse osmosis membrane.

According to the present invention, the energy and costs associating with the manufacturing of purified lactic acid are reduced and, at the same time, the heat deterioration and optical isomerization of lactic acid can be reduced. The problems, structure and effects described other than above will be apparent from the following description of preferred embodiments.

BRIEF DESCRIPTION OF THE DRAWINGS

FIG. 1 is a graph showing the solubility of calcium lactate.
FIG. 2 is a diagram showing the saccharification process of the present invention.
FIG. 3 is a diagram showing the fermentation process and the subsequent concentration step of the present invention.
FIG. 4 is a diagram showing an embodiment of the reverse osmosis membrane concentration step.
FIG. 5 is a diagram showing the purification process of the present invention.

DETAILED DESCRIPTION OF THE PREFERRED EMBODIMENTS

The present invention provides a manufacturing process of purified lactic acid.

The manufacturing process of purified lactic acid is roughly divided into three processes including the saccharification process, the fermentation process and the purification process.

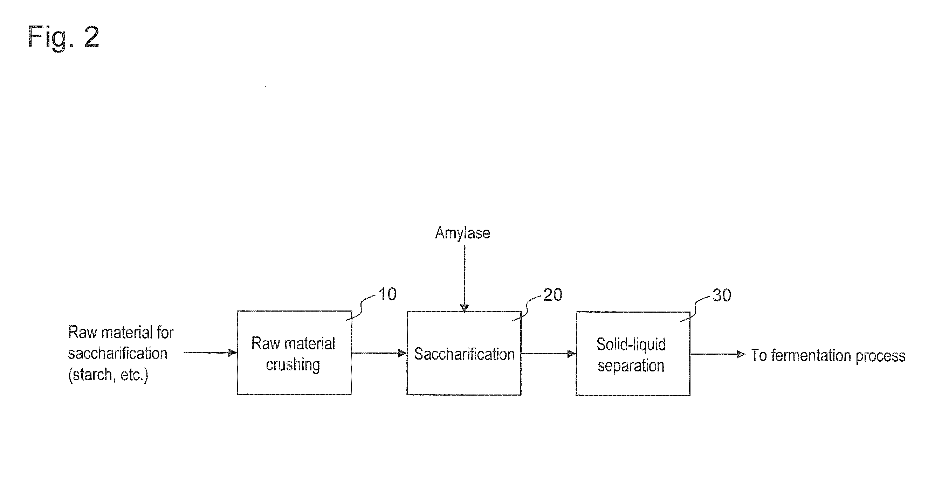

The saccharification process is a step of converting a saccharification raw material containing a carbon source, a nitrogen source and other nutrients to a sugar suitable for the lactic acid fermentation. Examples of the saccharification raw material include starches, such as cornstarch and potato starch, raw garbage containing amylose, etc. These saccharification raw materials, as shown in FIG. 2, are crushed in a crusher, etc. to fragment into small pieces in raw material crushing step 10.

In the saccharification step 20, amylase, one of the digestive enzymes, is added to the fragmented saccharification raw materials and the temperature is maintained at about 40°C to about 60°C. This procedure hydrolyzes the saccharification raw materials which are then converted to polysaccharides, such as glucose, maltose and oligosaccharide. Amylase can be industrially obtained as the product of microorganisms, such as *Aspergillus oryzae* and *Bacillus subtilis.*

The obtained solution containing polysaccharides contains impurities other than the polysaccharides. The solution, in the solid-liquid separation step 30, is subjected to a solid-liquid separation by centrifugal separation, or the like, and the solid contents, such as remaining starches and amylose, are removed. After decanting to remove the oil slick, the liquid chromatography treatment is carried out to produce a solution mainly containing polysaccharides.

Instead of producing the solution containing polysaccharides, syrups, such as saccharose, beet sugar and molasses, can also be used as the raw material.

Examples of the preferable raw material for lactic acid fermentation include, other than fermentation media containing the polysaccharides obtained in the saccharification process, recycled streams containing the lactate materials obtained during the manufacturing process of polylactic acid, or recycled polylactic acid hydrolyzed to prepare the solution containing lactate materials (for example, post-consumer waste collected from consumers or waste produced in the course of manufacturing.)

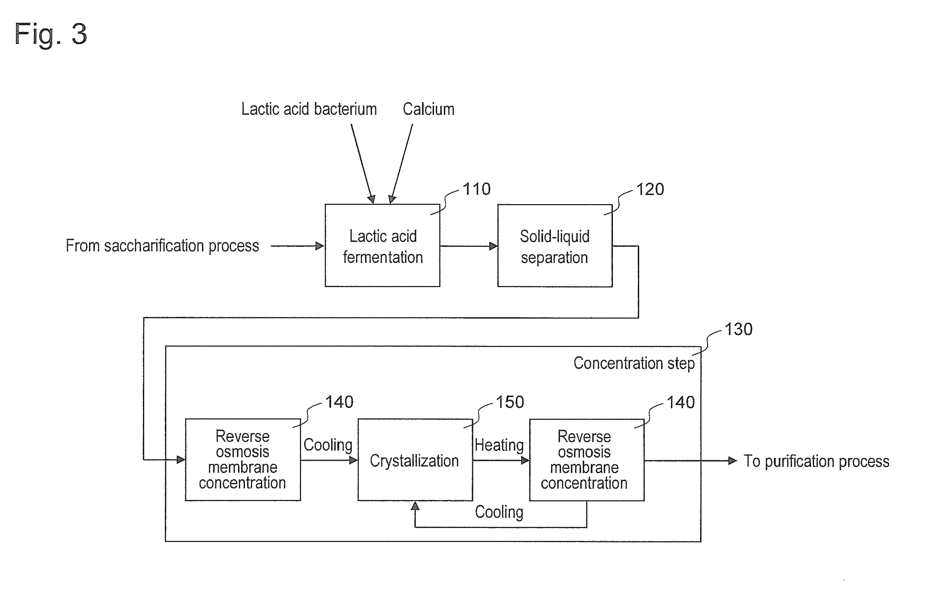

Next, the fermentation process is described. As shown in FIG. 3, in the lactic acid fermentation step 110, lactic acid or lactate is produced in the solution by fermentation using sugars as raw materials. In this step, the sugar concentration is typically 10 to 20% by weight. In the present specification, the "fermentation" refers to a metabolism by the microorganism culture. For the fermentation, microorganisms, such as bacteria and yeasts, are used. The fermented lactic acid liquid contains 2-hydroxypropionic acid in the form of either free acid or free acid salt and a lactic acid oligomer in the form of free acid or free acid salt. The terms "lactic acid" and "free lactic acid" as used in the present specification have the same meaning and, for example, refer to 2-hydroxypropionic acid and lactic acid oligomer in the form of acid. The salt form of lactic acid is lactate, specifically means sodium or calcium salt of lactic acid, etc. as well as the salt form of lactic acid oligomer.

The fermentation can be carried out using microorganisms, such as bacteria, fungi and yeasts, which are capable of producing lactic acid by the metabolism. Such microorganisms are known and bacteria belonging to *Lactobacillus* are typically used. For the fungus, those belonging to *Rhizopus* are used. For preferable yeasts, those belonging to Genus *Saccharomyces,* such as *Saccharomyces cerevisiae,* are used.

The fermentation is usually carried out at a temperature suitable for the specific microorganism used, in the temperature ranges of typically about 30°C to about 60°C for the bacteria fermentation, and typically about 20°C to about 45°C for the yeast fermentation. For the fungus fermentation, the temperature range is wide but most often within the range of about 25°C to about 50°C.

In the lactic acid fermentation step 110, the pH reduction associated with the lactic acid formation may sometimes cause decreased function of microorganisms. To prevent this, a hydroxide of alkali metal or alkaline earth metal (calcium), calcium carbonate, milk of lime, ammonia water or ammonia gas, or the like, is generally added as a pH adjusting agent to maintain the neutrality. As a pH adjusting agent is added, the cation of the pH adjusting agent binds to dissociated lactic acid whereby lactate is formed. Particularly, in the present invention, a calcium-containing pH adjusting agent, specifically a calcium salt, such as calcium carbonate, or calcium hydroxide, etc., is added to the lactic acid fermentation solution.

A fermentation raw material is fermented by a microorganism and a calcium-containing pH adjusting agent is added thereto to obtain a lactic acid fermentation liquid containing calcium lactate. Typically, the lactic acid fermentation liquid contains compounds other than calcium lactate called impurities and hence a solid-liquid separation using a technique, such as centrifugal separation, is carried out in the solid-liquid separation step 120 to remove the solid content. Examples of the impurities include cellular debris, residual carbohydrates and nutrients. The concentration of acceptable impurities contained in the purified lactic acid after removing these impurities varies depending on the commercial application of purified lactic acid or the lactic acid concentration. The concentration of calcium lactate in the fermentation liquid depends on the type of manufacturing process; sugars are converted to calcium lactate in a yield of substantially 100% in the case of batch processing system, but in the continuous system (the fermentation liquid is continuously extracted and sugars are continuously fed), it is efficiently kept at a concentration of 3 to 6% by weight.

Next, in the concentration step 130, the lactic acid fermentation liquid from which the impurities have been removed is concentrated. The concentration step 130 is carried out before the purification process in which sulfuric acid is added. The concentration of lactic acid fermentation liquid enables the lactic acid yield at the time of purification to increase and, at the same time, an amount of the liquid to be treated at the purification process in a later stage to be reduced, whereby the energy costs required for the treatment throughout the entire purification process can be reduced.

Generally, the concentration step 130 can be carried out by vaporization, pervaporation or other methods capable of selectively separating the above lactate materials, but, when these techniques are used, the lactic acid fermentation liquid must be maintained at a high temperature because the water is evaporated directly from the solution. In this case, an enormous amount of energy is required to remove water and the optical purity of lactic acid is reduced, posing problems of adversely affecting the application to polymers. In the present invention, the reverse osmosis membrane concentration step 140, wherein the water is removed by allowing the solution containing calcium lactate to pass through the reverse osmosis membrane, is used, as necessary, in combination with the crystallization step 150. The shape of reverse osmosis membrane is not limited and can be flat membrane, hollow fiber membrane, etc.

When a concentration of the lactate material is high, it reaches the solubility of calcium lactate which precipitates from the solution. If calcium lactate precipitates in the reverse osmosis membrane concentration step 140, the fouling on the reverse osmosis membrane surface occurs, making it difficult to operate the reverse osmosis. Accordingly, in the process of the present invention, the above solution is preferably concentrated to the solubility limit of calcium lactate but not exceeding the limit thereof.

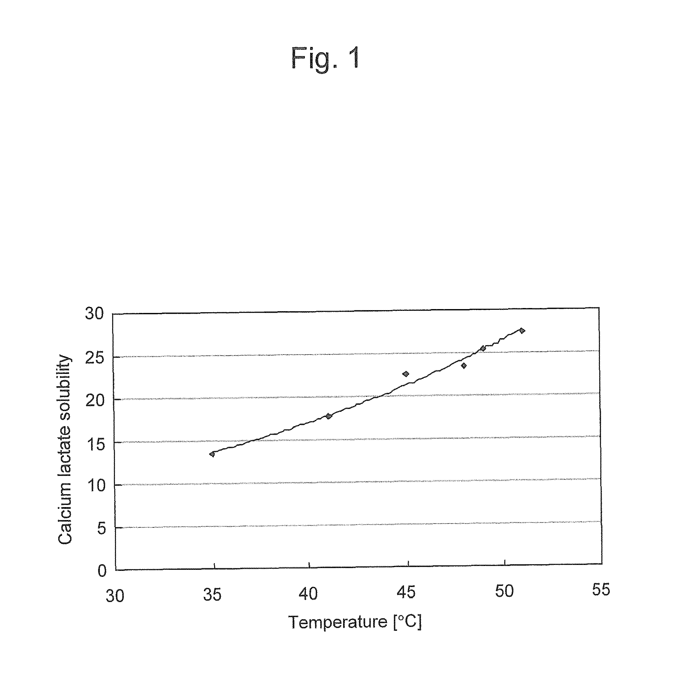

FIG. 1 shows the solubilities of calcium lactate at different temperatures measured by the present inventors. The measurements revealed that a solubility of about 15% by weight can be attained at about 40°C.

The desirable tolerance temperature of the most commercial reverse osmosis membranes is about 45°C or lower. In the present invention, the water is removed by a reverse osmosis membrane and the lactic acid fermentation liquid is concentrated at about 40°C, the temperature at which the fermentation of lactic acid fermentation liquid is completed.

Subsequently, the concentrated lactic acid fermentation liquid is cooled to a temperature at which the solubility of calcium lactate is low, for example, to 20°C. The solubility of calcium lactate at 20°C is about 5% by weight and thus calcium lactate of about 10% by weight, which is the difference from about 15% by weight of the solubility at 40°C, precipitates. In the crystallization step 150, the precipitate is separated and removed in the solid form.

A known method can be used to separate the crystallized calcium lactate from the solution. For example, the rotary drum vacuum filter and centrifuge can be used.

The concentration of calcium lactate solution after crystallized at about 20°C is about 5% by weight. When the solution is heated again to about 40°C at which the concentration is below the solubility of calcium lactate, the concentration by a reverse osmosis membrane can be carried out again as described above in the reverse osmosis membrane concentration step 140.

Then, as necessary, the solution is cooled again and, as shown in FIG. 3, the crystallization step 150 and the reverse osmosis membrane concentration step 140 are repeated to remove the water from the calcium lactate solution while avoiding the problem in occurrence of the fouling on the surface of reverse osmosis membrane, whereby the concentration step 130 in which calcium lactate is separated in the solid form is achieved. Assuming that the calcium lactate rejection rate by the reverse osmosis membrane is 100%, the three-step procedure of reverse osmosis membrane concentration at about 40°C, crystallization by cooling at about 20°C and reverse osmosis membrane concentration again by reheating to about 40°C can remove about 91% of the water contained in the lactic acid fermentation liquid, thereby reducing the water amount associated with the heating and concentrating step and the distillation purification step in the purification process in a later stage and whereby the energy costs required throughout the entire lactic acid manufacturing process can be reduced. For the calcium lactate rejection rate, when the calcium lactate concentrations in the liquid before and after the reverse osmosis membrane are represented as a and b respectively, the rejection rate $\chi$ is defined as

$$\chi = 1 - b/a$$

More specifically, the calcium lactate rejection rate 100% means that calcium lactate is not contained at all in the liquid which passed through the reverse osmosis membrane and mainly contains water.

The water removal process, which repeats the crystallization step 150 and the reverse osmosis membrane concentration step 140, is applicable, not only to calcium lactate, but generally to aqueous solutions containing a solid matter as the solute separable by crystallization. When the initial mass of an aqueous solution is represented as M [kg], the solute concentration is 100c [% by weight], the solubility of solute at a temperature T1 is 100a [% by weight] and the solubility of solute at a temperature T2 is 100b [% by weight] (herein hypothetically a < b), the solute contained in this aqueous solution is represented by Mc [kg] and water is M (1 - c) [kg]. Assuming that the rejection rate of a reverse osmosis membrane is 100%, the solute contained in the solution concentrated by a reverse osmosis membrane to the solubility limit at the temperature T2 is represented by Mc [kg] and water is Mc (1/b - 1) [kg]. Then, when the temperature of the obtained solution is lowered to T1, the solute in an amount equal to the solubility difference 100 (b - a) [% by weight] is crystallized. The water contained in the solution from which the crystallized solute is separated is represented as Mc (1/b - 1) [kg] and the solute is Mc (1/b - 1)/(1/a - 1) [kg] since it is determined by the solubility limit at the temperature T1. When the temperature of solution after the crystallization is brought back to the temperature T2 and concentrated by a reverse osmosis membrane to the solubility limit at the temperature T2, the solute contained in the concentrated solution is represented as Mc (1/b - 1)/(1/a - 1) [kg] and water is Mc (1/b - 1)$^2$/(1/a - 1) [kg].

Generally, in the case where a single-stage reverse osmosis membrane concentration is carried out and then an n-stage (provided that n ≥ 0) of the combination of crystallization and reverse osmosis membrane concentration is arranged, the final water amount contained in the concentrated solution is represented as Mc (1/b - 1)$^{n+1}$/(1/a - 1) [kg]. As the water amount of initial aqueous solution is M (1 - c) [kg], the water removal rate $R_n$ is represented by

$$R_n = 1 - \frac{(1/b - 1)^{n+1}}{(1/a - 1)^n} \cdot \frac{c}{1-c}$$

In the above example, c = 0.05, a = 0.05, b = 0.15 and n = 1, whereby $R_1$ = 0.911.

In the above example, the rejection rate $\chi$ = 1 is hypothetically given but, in the actual reverse osmosis membrane, at least a small amount of calcium lactate is found in the permeate and hence generally $\chi$ < 1. Thus, when a plurality of reverse osmosis membrane are linearly arranged to filter the permeate of a certain reverse osmosis membrane by another reverse osmosis membrane again, calcium lactate in the permeate can be collected and the reduction of lactic acid yield caused by the use of a reverse osmosis membrane can be prevented. Rejection rate $\chi$ is typically a function of the calcium lactate concentration and temperature of the treated liquid, however, given that $\chi$ = $\chi_0$ (constant value, $\chi_0$ < 1), in the case where the calcium lactate liquid having an initial concentration $C_0$ is filtered using a device with an n-stage reverse osmosis membranes linearly arranged, the final calcium lactate concentration $C_n$ in the liquid waste is

$$C_n = C_0 (1 - \chi_0)^n$$

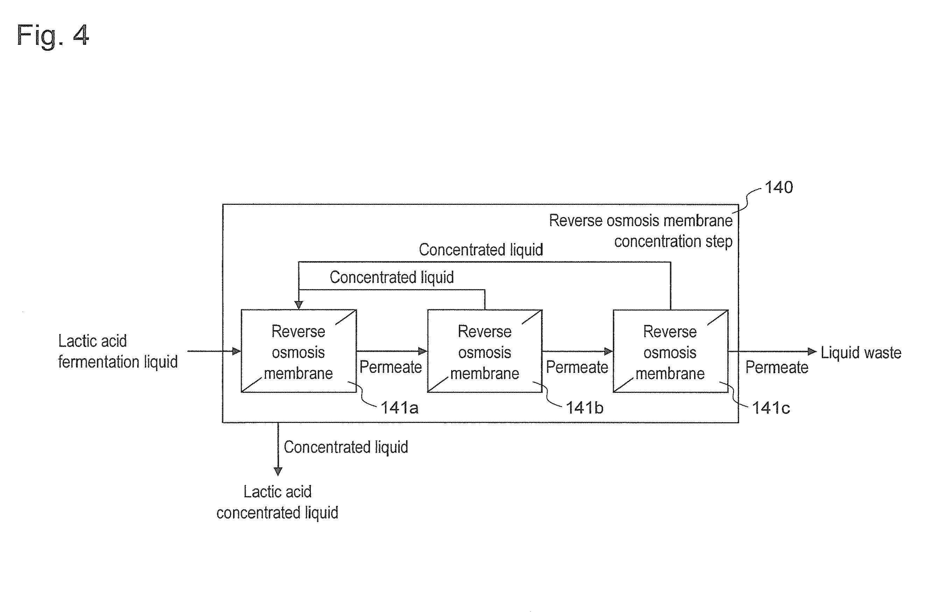

As an example shown in FIG. 4, in the case where the reverse osmosis membrane concentration step 140 is consisted of the structure with three-stage reverse osmosis membranes 141a to 141c linearly arranged, the concentration of calcium lactate contained in the final permeate can be reduced to 1/100 in comparison with the case of the only single-stage reverse osmosis membrane.

As described above, the lactic acid fermentation step is carried out typically at a temperature of about 20°C to 60°C. This temperature range is similar to the temperature 30°C to 60°C in the concentration step of the present invention. For this reason, according to the process of the present invention, the lactic acid fermentation liquid obtained in the lactic acid fermentation step, which is prior to the concentration step, is not required to be heated or cooled but can proceed to the concentration step without any treatment, which can reduce or eliminate the energy associated with the concentration. On the other hand, the concentration by means of the commonly practiced evaporation method requires a high temperature of 60°C to 150°C, which requires high energy costs for heating.

Further, since the lactic acid fermentation liquid after fermentation is not substantially required to be heated or cooled, the lactic acid fermentation liquid is free from unnecessary thermal history due to which the optical isomerization of lactic acid caused by thermal history can be reduced.

The lactic acid molecule has the chirality and the optical isomers in the form of L-isomer and D-isomer exist. The optical purity of lactic acid is critical in some industrial applications. The food application requires, as an example, 95% or higher L-isomer optical purity. In the microorganism fermentation, either one of the optical isomers is mainly produced. For example, *Lactobacillus delbrueckii* predominantly produces D-lactic acid, whereas *Lactobacillus casei* predominantly produces L-lactic acid. The optical purity of 95% means that 95% of the contained lactic acid or lactate is either one of the two optical isomers (L-isomer, D-isomer).

The optical purity of lactic acid affects the properties of polylactic acid. For example, the crystallizability of the above polymer is affected by the optical purity of the polymer. Specifically, the crystallization degree of the polymer affects its fabrication to polylactic acid resin fibers, nonwoven fabrics, films and other final products.

Accordingly, the optical purity of lactic acid is important in some applications, and the elimination of thermal history in the heating and concentrating and distillation steps inhibits the optical isomerization, obviating the possibility of lactic acid becoming unsuitable for specific chemical applications.

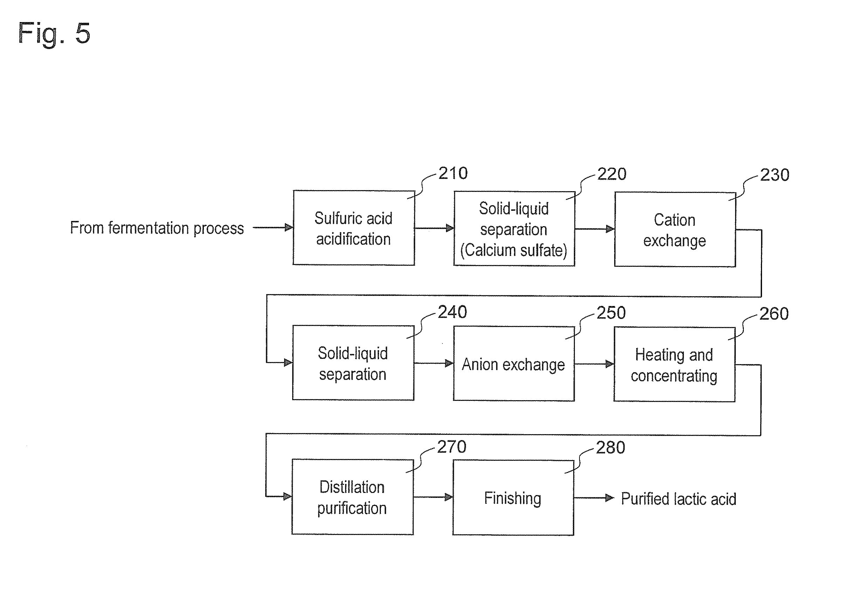

In the purification process, as shown in FIG. 5, the solution after the concentration step is first acidified (sulfuric acid acidification step 210), the lactic acid contained in the solution in the form of calcium lactate is converted from the dissociated form, i.e., the salt form, to the non-dissociated acid form. One of the methods for converting lactate to free lactic acid is to add a strong mineral acid, such as sulfuric acid, to the solution containing calcium lactate. The addition of sulfuric acid forms free lactic acid with calcium sulfate. Calcium sulfate is substantially water-insoluble and can be separated and removed easily by crystallization.

In the solid-liquid separation step 220 for calcium sulfate, known crystallization and filtration methods can be employed. For example, rotary drum vacuum filter and centrifuge can be used.

After removing the solid calcium sulfate, the impurity ions contained in the solution are removed in the desalination step. The desalination step can be carried out by various methods, such as ion exchange method, distillation, and solvent extraction. Hereinafter, the case of ion exchange method is described as an example.

Calcium ions remaining in the solution, from which calcium sulfate has been separated and removed, and the cations of metals, such as sodium, potassium and magnesium, contained as impurities in the fermentation raw materials are removed by cation exchange step 230. If necessary, a step of adsorbing microparticles using active carbon may be introduced before the cation exchange step. In the cation exchange step 230, the metal cation in the above lactic acid solution is removed by allowing it to contact with an ion exchange resin and substituting with hydrogen ion. An example of preferable cation exchange resin includes DIAION (trade name) made by Mitsubishi Chemical Corporation. The metal ion precipitated in the form of solid matter as a result of the cation exchange is subjected to a solid-liquid separation by sedimentation separation, or the like, in the solid-liquid separation step 240 to be removed from the solution.

The sulfate ion and organic acid anion, other than lactic acid, produced as by-products in the course of the fermentation and contained in the solution from which the metal ions have been removed can be removed by anion exchange step 250. The sulfate ion and organic acid anion are removed by allowing the sulfate ion and organic acid anion to contact with an anion exchange resin by which hydroxyl ion substitutes therefor. An example of preferable anion exchange resin is the DIAION mentioned earlier. About two to five ion exchange columns are desirably provided which are for both cation and anion exchanges. Through the present desalination step, the concentration of impurity ions in the solution can be reduced to 50 meq/L.

Next, in the heating and concentrating step 260, the solution from which the impurity ions have been removed is concentrated with heating to remove the water. For the heating and concentrating, techniques, such as reduced pressure and centrifugal thin film, can be used. In the heating and concentrating step 260, the temperature is desirably about 60°C to about 150°C and the pressure is desirably about 4 kPa to 10 kPa. The lactic acid solution of 80% by weight can be obtained by the heating and concentrating step 260.

After the heating and concentrating, the organic impurities are further removed by the distillation purification step 270. For the distillation, known means, such as distillation column, can be used. In the case where a distillation column is used, the distillation temperature is desirably about 60°C to 130°C and the distillation pressure is desirably about 500 Pa to 2000 Pa. The distillation column may be used more than one and about 1 to 5 columns are commonly used. The lactic acid solution of 90% by weight can be obtained by the distillation purification step 270. In the present invention, the lactic acid concentration is maintained at, for example, about 37% by weight by the concentration using reverse osmosis membranes. For this reason, the water removal amount by the heating and concentrating and the distillation is about 9% of the case wherein the reverse osmosis membrane concentration is not used, whereby the energy consumption saving is realized.

The distillation purification step 270 requires high-temperature treatment and optical isomers, even in a small amount, are produced due to the thermal history during the distillation. Also, as the impurities contained in the solution deteriorate by heat, the color tone of the solution gets darker in some cases. To solve this, the technique as described below can be used as the finishing step 280. Specifically, the formed optical isomers can be removed using an ultrafiltration membrane. For example, when the solution are allowed to successively pass through ultrafiltration membranes having a diameter of 1 to 2 μm and a diameter of 0.2 to 0.5 μm, 99% or higher optical purity can be obtained. To deal with the thermal deterioration of impurities, an additional separation step using active carbon, ion exchange resin or the like is carried out as necessary for the purpose of reducing the coloration. In accordance with the purpose of use, if necessary, distillation, liquid chromatography, or the like, may be carried out for further purification or concentration.

FIGS. 2 to 5 show one example of the preferred processes. In this process, a saccharification raw material, such as starch, is first crushed in the raw material crushing step 10, amylase is added thereto to saccharify in the saccharification step 20 and the unreacted starch is removed in the solid-liquid separation step 30. In the subsequent lactic acid fermentation step 110, lactic acid bacterium and calcium hydroxide as a pH adjusting agent are added to the obtained sugar-containing solution, thereby obtaining by fermentation a lactic acid fermentation liquid containing lactic acid in the form of calcium lactate. The solid content contained in the lactic acid fermentation liquid is separated in the solid-liquid separation step 120 and the water is removed in the concentration step 130. In the concentration step 130, the water is initially removed in the reverse osmosis membrane concentration step 140, the obtained concentrated solution is cooled, and calcium lactate is separated in the form of solid in the crystallization step 150. The solution part is heated and concentrated again in the reverse osmosis membrane concentration step 140. In the concentration step 130, the crystallization step 150 and the reverse osmosis membrane concentration step 140 are repeated as necessary. In the reverse osmosis membrane concentration step 140, in the case where the reverse osmosis membrane has a low rejection rate and calcium lactate leaks out into the permeate, the reverse osmosis membrane concentration step 140 can be configured to have reverse osmosis membranes 141a to 141c in a multistage arrangement where the solution passed through the reverse osmosis membrane 141a can be filtered again through the reverse osmosis membranes 141b and 141c provided downstream in order to reduce the amount of calcium lactate in the liquid waste which has been subjected to the reverse osmosis membrane concentration step 140 and thus prevent a reduction in the yield of lactic acid. The obtained concentrated solution of calcium lactate, in the sulfuric acid acidification step 210, is subjected to the crystallization of the calcium ion in the form of calcium sulfate, which is separated in the solid-liquid separation step 220. Next, the above acidified solution is allowed to pass through the cation exchange step 230 to convert metal ions derived from the fermentation raw materials, such as sodium and magnesium, and calcium ion left unremoved by the precipitation to metal salts, which are removed in the solid-liquid separation step 240. Further, in the anion exchange step 250, the organic acid ions caused by the impurities are removed, and through the heating and concentrating step 260 and the distillation purification step 270 by distillation columns, purified lactic acid solution of 90% by weight is obtained. The optical isomers contained in the obtained purified lactic acid solution are removed by the finishing step 280, whereby the purified lactic acid solution having an optical purity of 99.5% or higher can be obtained.

EXAMPLE

Example 1

An aqueous glucose solution as a raw material was put into a fermenter together with a bacterium belonging to genus *Lactobacillus* for the fermentation. Calcium hydroxide as a pH adjusting agent (neutralizer) was added to the fermenter to maintain a pH of 6.2 to 6.8. The fermentation temperature was set to 52°C and the fermentation was allowed to continue for 72 hours.

The concentration of lactic acid in the obtained lactic acid fermentation liquid was, when measured on a calcium lactate concentration basis, 5% by weight.

The above lactic acid fermentation liquid was subjected to the water removal using the reverse osmosis membrane (Duratherm HWS RO HR, manufactured by GE Water). The liquid to be treated may be maintained at a temperature of 30°C to 60°C, desirably 40°C to 50°C. In the present Example, the temperature was maintained at 40°C, which is the same as the fermentation temperature. As a result, the lactic acid fermentation liquid was concentrated to 15% by weight, which is the saturated concentration of calcium lactate at about 40°C. At the time, the rejection rate of the reverse osmosis membrane (Duratherm HWS RO HR, manufactured by GE Water) against calcium lactate at 40°C was about 90%. For this reason, the permeate was filtered and concentrated by the other reverse osmosis membranes, whereby the concentration of calcium lactate in the liquid waste was reduced to 1% of the stock solution. As a result, 72% of the water in the lactic acid fermentation liquid was removed. Subsequently, the concentrated liquid was cooled to 20°C to crystallize calcium lactate as a solid. The solid obtained had a 10% by weight of the lactic acid fermentation liquid before the concentration step. When the solution containing calcium lactate left after crystallization was heated again to 40°C and concentrated again by the reverse osmosis membranes, the water of 19% by weight of the lactic acid fermentation liquid before the concentration step was removed. The water of about 91 % was removed from the lactic acid fermentation liquid through a series of the concentration steps.

A 98% sulfuric acid solution was added to the obtained concentrated liquid and whereby the calcium ion in the solution was crystallized in the form of calcium sulfate. The calcium sulfate crystal was separated by centrifugal separation. The solubility of calcium sulfate at 42°C is 3 g/L, and the calcium sulfate exceeding the solubility can be separated as a solid.

The acidified lactic acid solution from which the calcium sulfate solid has been separated was subjected to the cation exchange treatment and metal salts were removed therefrom by centrifugal separation. Sulfate and organic acid salts were removed by centrifugal separation after the following anion exchange column. Owing to these ion exchange steps, residual calcium ion, cations, such as fermentation raw material-derived sodium, potassium, and magnesium, sulfuric acid ion and organic acid ion produced as impurities during the fermentation were reduced to 50 meq/L.

The solution after the ion exchange treatment was concentrated with heating at 100°C and 10 kPa, thereby obtaining a lactic acid solution of 80% by weight.

The concentrated lactic acid solution was distilled at 130°C and 1 kPa to remove the impurities, thereby also obtaining a lactic acid solution of 90% by weight.

The obtained lactic acid solution of 90% by weight was allowed to successively pass through ultrafiltration membranes having a diameter of 2 $\mu$m and a diameter of 0.5 $\mu$m, thereby obtaining an optical purity of 99.5%.

The present invention is not limited to the above embodiments and encompasses various modifications. For example, a part of the embodiment structure may be deleted, or substituted with other structures, or other structures may be added.

Specifically, for example, the concentration step 130 of FIG. 3 is consisted only of the reverse osmosis membrane concentration steps 140 by omitting the crystallization step 150, and the reverse osmosis membrane concentration steps 140 are provided with a multistage reverse osmosis membranes 141a to 141c as shown in FIG. 4, whereby the solution passed through the upper stage reverse osmosis membrane is allowed to pass again through the lower stage reverse osmosis membrane, thereby increasing a yield of calcium lactate.

The above embodiments of the invention as well as the appended claims and figures show multiple characterizing features of the invention in specific combinations. The skilled person will easily be able to consider further combinations or sub-combinations of these features in order to adapt the invention as defined in the claims to his specific needs.

DESCRIPTION OF SYMBOLS

| | |
|---|---|
| 10 | Raw material crushing step |
| 20 | Saccharification step |
| 30 | Solid-liquid separation step |
| 110 | Lactic acid fermentation step |
| 120 | Solid-liquid separation step |
| 130 | Concentration step |
| 140 | Reverse osmosis membrane concentration step |
| 141a to 141c | Reverse osmosis membranes |
| 150 | Crystallization step |
| 210 | Sulfuric acid acidification step |
| 220 | Solid-liquid separation step |
| 230 | Cation exchange step |
| 240 | Solid-liquid separation step |
| 250 | Anion exchange step |
| 260 | Heating and concentrating step |
| 270 | Distillation purification step |
| 280 | Finishing step |

**Claims**

1. A manufacturing process of purified lactic acid comprising a fermentation process where a pH adjusting agent containing calcium and a microorganism are added to a sugar-containing solution to produce lactic acid in the form of calcium lactate, and a purification process where sulfuric acid is added to a solution containing calcium lactate to separate calcium ions in the form of calcium sulfate,
wherein prior to the step (210) of adding sulfuric acid, a multistage concentration means comprising reverse osmosis memberanes (141a - 141c) is provided for the solution containing calcium lactate, wherein the solution passed through an upper stage reverse osmosis membrane (141a, 141b) is allowed to pass again through a lower stage reverse osmosis membrane (141b, 141c), to increase an amount of the calcium lactate to be collected.

2. A manufacturing process of purified lactic acid comprising a fermentation process where a pH adjusting agent containing calcium and a microorganism are added to a sugar-containing solution to produce lactic acid in the form of calcium lactate, and a purification process where sulfuric acid is added to a solution containing calcium lactate to separate calcium ions in the form of calcium sulfate,
wherein the manufacturing process further comprises, prior to the step (210) of adding sulfuric acid, a reverse osmosis membrane concentration step (140) where the solution containing calcium lactate is allowed to pass through the reverse osmosis membrane (141a - 141c) to remove water; a crystallization step (150) where the obtained concentrated liquid of calcium lactate is cooled to crystallize and remove calcium lactate; and a reverse osmosis membrane concentration step (140) where the solution from which calcium lactate has been removed is heated and allowed to pass through the reverse osmosis membrane (141a - 141c) to remove water, the crystallization step (150) and the subsequent reverse osmosis membrane concentration step (140) being repeated one or more times.

3. The manufacturing process of purified lactic acid according to Claim 2, wherein in the reverse osmosis membrane concentration step (140), a multistage concentration means comprising reverse osmosis membranes (141a - 141c) is provided for the solution containing calcium lactate, wherein the solution passed through an upper stage reverse osmosis membrane (141a, 141b) is allowed to pass again through a lower stage reverse osmosis membrane (141b, 141c), to increase an amount of the calcium lactate to be collected.

4. The manufacturing process of purified lactic acid according to Claim 1, wherein the calcium lactate-containing solution allowed to pass through the reverse osmosis membrane (141a - 141c) has a temperature of 30°C to 60°C.

5. The manufacturing process of purified lactic acid according to Claim 2, wherein the calcium lactate-containing solution allowed to pass through the reverse osmosis membrane (141a - 141c) has a temperature of 30°C to 60°C.

6. The manufacturing process of purified lactic acid according to Claim 3, wherein the calcium lactate-containing solution allowed to pass through the reverse osmosis membrane (141a - 141c) has a temperature of 30°C to 60°C.

# Fig. 1

Fig. 2

# Fig. 3

EP 2 562 263 A1

Fig. 4

EP 2 562 263 A1

Fig. 5

From fermentation process →

Sulfuric acid acidification `210`

→ Solid-liquid separation (Calcium sulfate) `220`

→ Cation exchange `230`

Solid-liquid separation `240`

→ Anion exchange `250`

→ Heating and concentrating `260`

Distillation purification `270`

→ Finishing `280`

→ Purified lactic acid

EUROPEAN SEARCH REPORT

Application Number

EP 12 18 0784

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 01/25180 A1 (CARGILL DOW LLC [US]; MIZRAHI JOSEPH [IL]; EYAL AHARON M [IL]; RIKI CA) 12 April 2001 (2001-04-12) <br> * page 2, lines 20-29; figure 1 * <br> * page 5, line 30 - page 6, line 5 * <br> * page 22, line 5 - page 23, line 29; examples 4-5 * | 1-6 | INV. <br> C12P7/56 |
| Y | US 2011/028759 A1 (LUM OOI LIN [SG] ET AL) 3 February 2011 (2011-02-03) <br> * page 1, paragraph [0007] * <br> * claims 1,19-21 * <br> * page 2, paragraph [0023] - page 3, paragraph [0030] * <br> * page 4, paragraph [0043] - page 5, paragraph [0047]; figures 1-7; examples 1-10 * | 1-6 | |
| Y | CHU, H.C. ET AL.,: "High-temperature reverse osmosis membrane element", DESALINATION, [Online] vol. 70, 1988, pages 65-76, XP002687188, Retrieved from the Internet: URL:http://ac.els-cdn.com/0011916488850446 /1-s2.0-0011916488850446-main.pdf?_tid=b06 3ae76-2e73-11e2-a2f1-00000aab0f01&acdnat=1 352908699_99d98b75f254631922536cff4d618e9d > [retrieved on 2012-11-14] * page 65, paragraph 1 * | 1-6 | |
| A | DATABASE WPI Week 200921 Thomson Scientific, London, GB; AN 2009-F15301 XP002687189, & JP 2009 034030 A (TORAY IND INC) 19 February 2009 (2009-02-19) * abstract * | 1-6 | |

TECHNICAL FIELDS SEARCHED (IPC)

C12P

The present search report has been drawn up for all claims

| Place of search | Date of completion of the search | Examiner |
|---|---|---|
| Berlin | 14 November 2012 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 18 0784

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2012

| Patent document cited in search report | | Publication date | Patent family member(s) | | Publication date |
|---|---|---|---|---|---|
| WO 0125180 | A1 | 12-04-2001 | AU | 1190601 A | 10-05-2001 |
| | | | BR | 0014510 A | 11-06-2002 |
| | | | CN | 1377335 A | 30-10-2002 |
| | | | EP | 1220828 A1 | 10-07-2002 |
| | | | HK | 1048985 A1 | 02-09-2005 |
| | | | JP | 2003511360 A | 25-03-2003 |
| | | | KR | 20020040833 A | 30-05-2002 |
| | | | US | 2002102672 A1 | 01-08-2002 |
| | | | US | 2003004375 A1 | 02-01-2003 |
| | | | WO | 0125180 A1 | 12-04-2001 |
| | | | ZA | 200203508 A | 30-07-2003 |
| US 2011028759 | A1 | 03-02-2011 | AU | 2010202280 A1 | 17-02-2011 |
| | | | CA | 2706205 A1 | 28-01-2011 |
| | | | CN | 101967091 A | 09-02-2011 |
| | | | SG | 168460 A1 | 28-02-2011 |
| | | | US | 2011028759 A1 | 03-02-2011 |
| JP 2009034030 | A | 19-02-2009 | NONE | | |

EPO FORM P0459

For more details about this annex : see Official Journal of the European Patent Office, No. 12/82

**REFERENCES CITED IN THE DESCRIPTION**

**Patent documents cited in the description**

- WO 01025180 A